(19) Europäisches Patentamt

European Patent Office

Office européen des brevets

(11) **EP 1 517 731 B1**

(12) **EUROPÄISCHE PATENTSCHRIFT**

(45) Veröffentlichungstag und Bekanntmachung des Hinweises auf die Patenterteilung:
**30.08.2006 Patentblatt 2006/35**

(21) Anmeldenummer: **03732446.4**

(22) Anmeldetag: **23.05.2003**

(51) Int Cl.:
**B01D 9/00** *(2006.01)*     **C07C 317/46** *(2006.01)*
**A01N 41/10** *(2006.01)*

(86) Internationale Anmeldenummer:
**PCT/EP2003/005395**

(87) Internationale Veröffentlichungsnummer:
**WO 2003/099409 (04.12.2003 Gazette 2003/49)**

(54) **VERFAHREN ZUR HERSTELLUNG EINER SPEZIFISCHEN KRISTALLMODIFIKATION VON 2-(2-CHLOR-4-MESYL-BENZOYL)-CYCLOHEXAN-1,3-DION**

METHOD FOR PRODUCING A SPECIFIC CRYSTALLINE MODIFICATION OF 2-(2-CHLOR-4-MESYL-BENZOYL)-CYCLOHEXANE-1,3-DIONE

PROCEDE DE REALISATION D'UNE MODIFICATION CRISTALLINE SPECIFIQUE DE 2-(2-CHLOR-4-MESYL-BENZOYL)-CYCLOHEXANE-1,3-DIONE

(84) Benannte Vertragsstaaten:
**AT BE BG CH CY CZ DE DK EE ES FI FR GB GR HU IE IT LI LU MC NL PT RO SE SI SK TR**

(30) Priorität: **29.05.2002 DE 10223913**

(43) Veröffentlichungstag der Anmeldung:
**30.03.2005 Patentblatt 2005/13**

(73) Patentinhaber: **Bayer CropScience Aktiengesellschaft**
**40789 Monheim (DE)**

(72) Erfinder:
• EBLE, Axel
  **50668 Köln (DE)**
• SIRGES, Wolfram
  **40597 Düsseldorf (DE)**
• SCHWIEDOP, Ulrich
  **40789 Monheim (DE)**
• HEYN, Armin
  **51467 Bergisch Gladbach (DE)**

(56) Entgegenhaltungen:
**EP-A- 0 137 963        WO-A-01/10830**
**WO-A-01/64672**

**Beschreibung**

Die vorliegende Erfindung betrifft ein Verfahren zur Herstellung einer spezifischen Kristallmodifikation einer polymorphen, organischen Substanz durch Fällung der spezifischen Kristallmodifikation aus dem in einer wässrigen Lösung vorliegenden Salz der polymorphen Substanz unter Verwendung üblicher wasserlöslicher organischer Lösungsmittel als Additive sowie einer Säure oder Base.

Die Polymorphie von Wirkstoffen ist von großer Bedeutung für die chemische Entwicklung, die Verfahrensentwicklung und die Entwicklung von Formulierungen. Es ist bekannt, dass einige organische Verbindungen in nur einer Kristallstruktur, andere (so genannte Polymorphe) in zwei oder mehr Strukturen (ebenso Modifikationen oder Kristallmodifikationen genannt) vorkommen können. Es ist nicht möglich, die Zahl der Kristallmodifikationen einschließlich ihrer physikalisch-chemischen Eigenschaften vorherzusagen, besonders ihre thermodynamische Stabilität, wie auch das unterschiedliche Verhalten nach Darreichung in lebenden Organismen.

Es ist bekannt, dass für einige Polymorphe eine bestimmte Modifikation über den gesamten Temperaturbereich bis zum Schmelzpunkt die thermodynamisch stabile Phase darstellt, wohingegen bei anderen Stoffsystemen ein oder mehrere Übergangspunkte existieren, bei dem sich das Stabilitätsverhältnis umkehrt. In einem Bereich abseits dieser Übergangspunkte ist immer eine bestimmte Modifikation thermodynamisch stabil. Alle anderen Modifikationen, die in diesem Bereich existieren, sind metastabil und wandeln sich früher oder später in die thermodynamisch stabile Modifikationen um. Die Zeit innerhalb der sich eine solche Umwandlung vollzieht ist stoffspezifisch und hängt von der Kinetik des Stoffsystems ab. Die stoffspezifische Kinetik bestimmt auch darüber welche Modifikation sich aus einem hristallisations- oder Fällprozess heraus bildet. Es ist nicht möglich, das Stabilitätsverhältnis koexistenter Kristallmodifikationen insbesondere die Existenz und Lage der oben bezeichneten Übergangspunkte vorherzusagen. Es ist weiterhin weder möglich die Kinetik der Umwandlung von metastabilen zu stabileren Modifikationen vorherzusagen, noch welche Modifikation sich aus einem Kristallisations- oder Fällprozess heraus bildet. Ein aktueller Überblick über den Stand des Wissens zu diesen prinzipiellen thermodynamischen und kinetischen Verhältnissen ist in J. Bernstein, R.J. Davey, J.O. Henck, Angew. Chem. Int. Ed., 1999, 38, 3440-3461 wiedergegeben.

Es ist bekannt, dass die Bildung metastabiler Modifikationen bevorzugt sein kann, obwohl die Triebkraft für die Bildung der thermodynamisch stabilen Phase aus einem Kristallisations- oder Fällprozess am größten ist. Da die Zeit innerhalb der sich eine Umwandlung lcoexistenter Kristallmodifikationen vollzieht üblicherweise um Größenordnungen länger ist als die Zeit der Phasenbildung aus einem Kristallisations- oder Fällprozess, verbleiben diese Produkte, die in einer metastabilen Struktur gebildet wurden, während des weiteren Herstell- und Verarbeitungsprozess üblicherweise zunächst in diesem Zustand. Einige bekannte Beispiele für dieses Verhalten sind in J. Bernstein et al. angegeben (siehe oben). Zahlreiche Probleme können vor allem bei Wirkstoffen durch die Verwendung oder Verarbeitung metastabiler Kristallmodifikationen hervorgerufen werden. Diese Probleme, verursacht im Wesentlichen durch unerwünschtes und unkontrolliertes Kristallwachstum (Rekristallisation), können bei der Herstellung, der Formulierung, der Lagerung, dem Transport, oder in der Anwendung in Erscheinung treten und können signifikante Änderungen der Bioverfügbarkeit, Verbackungen usw. bewirken (J. Halebian, W. McCrone, J. Pharm. Sci. 58 (1969) 911).

Ein Beispiel für solche Probleme ist 2-(2-Chlor-4-mesyl-benzoyl)-cyclohexan-1,3-dion, im Weiteren Sulcotrione genannt, welches herbizide Eigenschaften besitzt und zur Herstellung von Unkrautbekämpfungsmitteln eingesetzt werden kann. Sulcotrione tritt zum Beispiel in zwei Modifikationen in Erscheinung (vgl. dazu die deutsche Patentanmeldung mit der Anmeldenummer 10152459.5, die als Ganzes Teil der vorliegenden Amneldung sein soll). Die metastabile Modifikation, im Weiteren Modifikation (I) genannt, ist das Produkt eines Herstellprozesses, der der Beschreibung in EP-A2-0 186 117 folgt. Da diese Modifikation metastabil ist, ist sie im Vergleich zur stabilen Modifikation weniger für die Herstellung, die Formulierung, die Lagerung und die Applikation dieses Wirkstoffs geeignet.

Gemeinhin bekannte Methoden zur reproduzierbaren Herstellung der stabilen Modifikation einer Substanz sind die Verdampfungs- oder Kühlungskristallisation, wie auch die Rekristallisation in einem sorgfältig ausgewählten Lösungsmittel (vgl. WO 97/49681 A1). Die Wahl eines passenden Lösungsmittels erfolgt dabei mit dem Ziel, die Grenzflächenspannung des Kristalls mit der umgebenden Lösung und/oder die Komplexbildung der Moleküle im Zuge der Kristallisation zu beeinflussen, um die Bildung der gewünschten Kristallstruktur zu fördern (N. Bladgen, Powder Technology, 121 (2001) 46-52; R. J. Davey et al., Crystal Growth & Design, 1(1), (2001) 59-65; US 5,959,108; US 5,939,555). In WO 01/64672 AI wird z.B. ein Verfahren zur Gewinnung einer spezifischen Modifikation beschrieben, das auf der Lösung des Isomorphs in Butanon/Wasser (10:1) bei anschließender Zugabe einer Säure beruht. Der wesentliche Nachteil solcher Strategien ist, dass ein Lösungsmittel, welches auf diesem Weg für die Kristallisation gefunden wurde, nur selten für die vorangehenden Verfahrenschritte und Reaktionsstufen geeignet ist. Als Folge dessen sind kostenintensive Maßnahmen wie z.B. der Wechsel des Lösungsmittels oder eine Rekristallisation einer bereits gebildeten metastabilen Kristallstruktur notwendig. Insbesondere bringen solche Verfahren meist auch einen signifikanten Verlust an Ausbeute mit sich.

Eine andere bekannte Methode, um gezielt auf die Bildung spezifischer Kristallstrukturen einer polymorphen Substanz Einfluss zu nehmen, ist die Verwendung so genannter "maßgeschneiderter" (engl.: "tailor-made") Additive (E.

Staab et al., Adv. Mater.,2 (1990) 40). Ziel solcher "maßgeschneiderten" Additive ist ebenfalls die Einflussnahme auf die Grenzflächenspannung des Kristalls zur umgebenden Lösung und/oder die Komplexbildung der Moleküle im Zuge der Kristallisation, um die Bildung der gewünschten Kristallstruktur zu fördern (vgl. z.B. US 5,716,445 A).

Charakteristisch für "maßgeschneiderte" Additive ist eine partielle Übereinstimmung oder Ähnlichkeit der Molekülstruktur zu der polymorphen Substanz. Der Vorteil einer Verwendung solcher "maßgeschneiderten" Additive im Vergleich zu der oben genannten Strategie ist die Möglichkeit einer Anwendung in unterschiedlichen Lösungsmitteln, was die Implementierung eines solchen Verfahrens einfacher macht. Die spezifisch gestalteten Additive müssen jedoch in aller Regel exklusiv für ihre Verwendung zur gezielten Beeinflussung der Polymorphie hergestellt werden, was ihre Entwicklung zeitaufwendig und die Verfahren, in denen die Additive verwendet werden, teuer macht.

Aufgabe der vorliegenden Erfindung war es deshalb, ein Verfahren zur Herstellung spezifischer Modifikationen von 2-(2-Chlor-4-mesyl-benzoyl)-cyclohexan-1,3-dion bereitzustellen, welches zur effizienten und preiswerten Gewinnung einer spezifischen Modifikation von 2-(2-Chlor-4-mesyl-benzoyl)-cyclohexan-1,3-dion genutzt werden kann. Insbesondere war es Aufgabe der vorliegenden Erfindung, ein Verfahren zur Gewinnung der Modifikation zugänglich zu machen, das ohne einen Wechsel des Lösungsmittels und/oder eine Rekristallisation auskommt.

In der vorliegenden Erfindung wurde die Aufgabe gelöst, indem preiswerte und in einfacher Weise einsetzbare Additive gemäß Anspruch 1 verwendet werden, um die Entstehung der spezifischen Kristallnodifikation zu erreichen. Diese Additive zeigen ihre Wirkung auch in Lösungsmitteln, die in den vorangehenden Reaktions- und Verfahrensschritten verwendet werden, sodass auf einen Wechsel des Lösungsmittels und/oder zusätzliche Verfahrensschritte verzichtet werden kann.

Es wurde nun gefunden, dass die organischen Lösungsmittehnoleküle gemäß Anspruch 1 hervorragend geeignete Additive zur Förderung der Ausbildung spezifischer Kristall-Strukturen organischer polymorpher Substanzen in Säure-/Base-Fällungskristallisationen sein können. Überraschenderweise gelingt die Herstellung spezifischer Modifikationen polymorpher organischer Substanzen direkt durch die Fällung der spezifischen Kristallmodifikation aus dem Salz der polymorphen Substanz in wässriger Lösung gemäß der folgenden Reaktionsgleichung,

$$\text{Salz der polymorphen Substanz} + \text{Säure oder Base} \rightarrow \text{Substanz mit spezifischer Kristallstruktur} + \text{korrespondierendes Salz}$$

wobei die Fällung der Substanz in der gewünschten Kristallstruktur durch Zugabe der organischen wasserlöslichen Lösungsmittel als Additive gefördert wird.

Der Begriff "Additiv", wie er hierin verwendet wird, bezieht sich auf Verbindungen bzw. Substanzen, die einer vorliegenden Lösung einer Substanz zugesetzt werden, wobei die Menge (w/w) des zugesetzten Additivs vorzugsweise kleiner ist als die Menge der in der Lösung vorliegenden Substanz. Vorzugsweise wird das Additiv in einer Menge von 0,1 bis 20 % bezogen auf die Menge der zu fällenden Substanz eingesetzt, besonders bevorzugt in einer Menge von 1 bis 10 % und insbesondere von 1 bis 5%. Es ist dabei unerheblich, ob das Additiv der vorliegenden Lösung zugesetzt oder besagte Lösung einem vorliegendem Additiv bzw. einer Lösung enthaltend das Additiv zugesetzt wird.

Gegenstand der vorliegenden Erfindung ist deshalb ein Verfahren gemäß Anspruch 1

Säuren und Basen, die im erfindungsgemäßen Verfahren verwendet werden können, sind die in der organischen Chemie üblicherweise eingesetzten starken Säuren oder Basen. Zu den verwendbaren Säuren gehört z.B. HCl, $H_2SO_4$, $HNO_3$ oder HF. Zu den verwendbaren Basen gehört z.B. NaOH, $Ba(OH)_2$, $Ca(OH)_2$ oder KOH.

Organische wasserlösliche Lösungsmittel, die in diesem Verfahren zur gezielten Ausbildung der gewünschten Modifikation als Additive eingesetzt werden können, sind unter anderem kurzkettige Alkohole, wie z.B. Methanol, Ethanol oder 2-Propanol, kurzkettige Ketone, wie z.B. Aceton oder 2-Butanon, Als niedere bzw. kurzkettige Alkohole werden hier solche Alkohole bezeichnet, die eins bis zehn Kohlenstoffatome aufweisen, bevorzugt eins bis fünf Kohlenstoffatome. Als niedere bzw. kurzkettige Ketone werden hier solche Ketone bezeichnet, die drei bis zehn Kohlenstoffatome aufweisen, bevorzugt drei bis fünf Kohlenstoffatome.

Die Wirkweise dieser Additive, welche die Ausbildung kinetisch unterdrückrter Modifikationen fördem, besteht darin, dass sie einen Bereich der Übersättigung eröffnen, innerhalb dessen die gewünschte Modifikationen hergestellt werden kann. Wenn dieser Bereich durch zu hohe Übersättigung überschritten wird, treten die kinetisch bevorzugten Modifikationen wieder in Erscheinung. Daher ist neben bzw. in Kombination mit der Verwendung von wasserlöslichen organischen Lösungsmitteln als Additive auch eine Fällungskristallisation bei kontrollierter Übersättigung Bestandteil dieser Erfindung. Gegenstand der Erfindung ist damit ein Verfahren zur Herstellung einer spezifischen Kristallmodifikation einer polymorphen Substanz durch Ausfällen der spezifischen Kristallmodifikation aus dem in einer wässrigen Lösung vorliegenden Salz der polymorphen Substanz bei geringer Übersättigung bei Anwesenheit einer Säure oder Base und eines wasserlöslichen, organischen Lösungsmittels.

Die Fällungskristallisation kann durchgeführt werden, indem eine korrespondierende (äquinormale) Menge einer Säure/Base zu einem gerührten Reaktionsgefäß gegeben wird, in das die wässrige Lösung des Salz des Wirkstoffs zusammen mit dem Additiv vorgelegt wurde. Vorzugsweise wird die Säure oder Base in geringem Überschuss zugegeben, um den erwünschten pH-Wert einzustellen. Gegebenenfalls werden dabei Impfkristalle der gewünschten Modifikation zugegeben, wenn die Lösung leicht übersättigt ist.

Gegenstand der vorliegenden Erfindung ist deshalb insbesondere ein Verfahren zur Herstellung einer spezifischen Kristallmodifikation der polymorphen Substanz wie vorstehend beschrieben, indem man die Säure oder Base zu einer Mischung aus der wässrigen Lösung des Salzes der polymorphen Substanz und eines geeigneten Additivs sowie gegebenenfalls Impfkristallen zugibt.

Die Fällungskristallisation kann ebenfalls durchgeführt werden, indem die wässrige Lösung des Salzes der polymorphen Substanz unter Rühren zu einer Mischung aus der korrespondierenden Menge einer Säure oder Base und einem geeigneten Additiv sowie gegebenenfalls Impfkristallen der gewünschten Modifikation zugegeben wird.

Bevorzugt wird die Fällungskristallisation durchgeführt, indem man die wässrige Lösung des Salzes der polymorphen Substanz und eine korrespondierende Menge einer Säure/Base parallel und gleichförmig unter Rühren zur wässrigen Lösung des Additivs und gegebenenfalls Impfkristallen der gewünschten Modifikation zugibt. Die Dosierung der jeweiligen zugegebenen Komponente erfolgt dabei so, dass der pH-Wert im Reaktionsansatz jeweils konstant gehalten werden kann.

Der pH-Wert, der im erfindungsgemäßen Verfahren verwendet wird, hängt von der jeweiligen polymorphen Substanz bzw. deren zugehöriger spezifischer Modifikation ab und kann über einen relativ großen Bereich variiert werden. Der optimale pH-Wert für eine Fällung kann in einfacher Weise bestimmt werden, indem die Ausbeute des erfmdungsgemäßen Verfahrens bei verschiedenen pH-Werten bestimmt und der pH-Wert mit der größtmöglichen Ausbeute ausgewählt wird.

Die Menge des erfindungsgemäßen Additivs, das im erfindungsgemäßen Verfahren eingesetzt wird, kann über einen größeren Bereich variiert werden. Bevorzugt werden weniger als zehn Prozent (w/w) des Additivs bezogen auf die Ausbeute an gefällter Substanz eingesetzt, besonders bevorzugt weniger als ein Prozent. Das Additiv kann jedoch auch in größeren Mengen als zehn Prozent zugegeben werden.

Die Menge der Impfkristalle, die gegebenenfalls im erfindungsgemäßen Verfahren verwendet wird, kann ebenfalls über einen größeren Bereich variiert werden. Bevorzugt werden weniger als zehn Prozent (w/w) bezogen auf die Ausbeute an gefällter Substanz eingesetzt, besonders bevorzugt weniger als ein Prozent. Es können jedoch auch mehr als zehn Prozent zugegeben werden. Das Verfahren kann auch ohne die Zugabe von Impfkristallen durchgeführt werden, wenn dass Additiv effizient genug ist.

Das Verfahren wird bevorzugt bei Normaldruck und bei Temperaturen kleiner 100°C durchgeführt. Besonders geeignet erweisen sich dabei Temperaturen bei denen die jeweils gewünschte Kristallmodifikation die stabile Form darstellt. Die geeignete Temperatur bzw. Übergangspunkte der gewünschten, spezifischen Kristalhnodifikation mit jeder anderen existierenden Modifikationen können in dem Fachmann bekannter Weise festgestellt werden. Beim Übergangspunkt liegt dabei die Temperatur vor, bei der zwei kristalline Phasen einer Substanz bei gegebenem Druck im Gleichgewicht sind. Zur Bestimmung des Übergangspunktes zwischen zwei bekannten Modifikationen kann z.B. die Löslichkeit beider Modifikationen getrennt voneinander in irgendeinem geeigneten Lösungsmittel in Abhängigkeit von der Temperatur bestimmt werden. Schnittpunkte der Löschchkeitskurven zweier Modifikationen in einem Lösungsmittel bei gegebenem Druck kennzeichnen die Temperatur, bei der ein Übergangspunkt des Stabilitätsverhältnisses zwischen den beiden Modifikationen besteht. Besonders geeignet ist im erfindungsgemäßen Verfahren die Verwendung von Temperaturen, die mehr als 10°C von Übergangspunkten der gewünschte Kristallmodifikation mit jeder anderen existierenden Modifikation entfernt sind, wobei Temperaturen, die am weitesten von allen Übergangspunkten der gewünschten Kristallmodifikation mit jeder anderen existierenden Modifikation entfernt sind, insbesondere bevorzugt werden. Das Verfahren kann auch bei Drücken abweichend vom Normaldruck und bei Temperaturen größer 100°C durchgeführt werden.

Die Dosierrate mit der die Säure/Base, oder mit der die wässrige Lösung des Salzes der polymorphen Substanz bzw. mit der beide dem Reah-tionsansatz unter Rühren zugegeben werden können, hängt ab von der Kinetik des Stoffsystems, von der Konzentration der Eduktlösungen, von der Menge der Impfkristalle und von der Effektivität des verwendeten Additivs. Die Dosierrate kann jedoch über einen größeren Bereich variiert und eine günstige Dosierrate in einfacher Weise ermittelt werden. Es ist dabei wichtig, die Dosierrate so zu wählen, dass es vor allem zu Beginn der Zugabe, z.B. der wässrigen Lösung des Salzes der polymorphen Substanz zur vorgelegten Säure oder Base, nicht zu unkontrollierter Übersättigung (Übersättigungsspitzen) kommt. Der kontrollierte Verlauf der Fällung wird durch Rühren des Ansatzes begünstigt.

Die Spanne der Löslichkeit der Verbindung zu seinem Salz umfasst idealerweise mehr als zwei Größenordnungen. So sollte die Löslichkeit des Salzes der polymorphen Ausgangsverbindung vorzugsweise größer als 10 Gew.-%, bevorzugt größer als 20 Gew.-% und besonders bevorzugt größer als 30 Gew.-% sein, während die Löslichkeit der erwünschten, spezifischen Kristallmodifikation bei einem optimalen pH-Wert wie vorstehend beschrieben idealerweise kleiner als 5 Gew.-%, bevorzugt kleiner als 1 Gew.-% und besonders bevorzugt kleiner als 0,1 Gew.-% sein sollte.

Im bereits vorstehend genannten Beispiel wird Sulcotrione mit Hilfe des erfindungsgemäßen Verfahrens die Fällung der Modifikation II von Sulcotrione aus seinem Natriumenolat durch Zugabe von Salzsäure erreicht werden.

Gegenstand des vorliegenden Verfahrens ist deshalb auch ein Verfahren zum Herstellen der Modifikation II von Sulcotrione, welches wie vorstehend beschrieben durchgeführt wird.

Wie vorstehend beschrieben, kann auch hier gemäß dem erfindungsgemäßen Verfahren neben dem Natrium-Ion jede andere korrespondierende Base zum Enolat-Anion eingesetzt werden. Ebenso kann das Verfahren mit jeder anderen korrespondierenden Säure als der bezeichneten Salzsäure durchgeführt werden. Das Verfahren kann ebenso auf der Fällung einer Base aus ihrem Salz durch Zugabe einer stärkeren Base beruhen. Das gewählte Beispiel Sulcotrione, bei dem die Fällung einer Säure aus ihrem Salz durch Zugabe einer stärkeren Säure erfolgt, ist also nicht limitierend auszulegen.

Bei Sulcotrione ist z.B. eine Temperatur von 12°C geeignet für die Fällung der Modifikation II, da die Temperatur ausreichend weit entfernt von den Übergangspunkten liegt und die Modifikation II in diesem Temperaturbereich die stabile Modifikation darstellt. Heterozyklische Amine zeigen sich ebenso wie kurzkettige Alkohole und Ketone als äußerst effiziente Additive für die Förderung der Ausbildung der Modifikation II.

Zur Herstellung der Modifikation II von Sulcotrione ergab z.B. die parallele Dosierung der wässrigen Natriumenolatlösung und der Salzsäure bei einem konstanten pH-Wert die besten Ergebnisse. Eine eventuelle Zugabe von Impfkristallen erfolgt vorzugsweise bei eintretender Übersättigung der Lösung. Für die parallele Dosierung wurde ein konstanter pH-Wert von 2,7 gewählt, da die Ausbeute bei diesem pH-Wert optimal ist. Da der Einfluss der Vermischung wie vorstehend bemerkt von großer Bedeutung bei der Fällungskristallisation ist, muss Sorge getragen werden, dass der Leistungseintrag des Rührers trotz eines rasch ansteigenden Füllvolumens im gewählten Reaktionsgefäß konstant gehalten wird. Ein moderater Leistungseintrag mit einem Impeller von etwa 0,15 W/kg in Kombination mit einer gegenüberliegenden Zugabe beider Edukte auf die Oberfläche der Vorlage ergibt ideale Bedingungen, um lokale Übersättigungsspitzen zu vermeiden.

### Beispiele

### Beispiel 1 - Vergleichsbeispiel

Herstellung der stabilen Kristallstruktur (Modifikation II) von 2-(2-Chlor-4-mesylbenzoyl)-cyclohexan-1,3-dion (Sulcotrione) durch direkte Fällung (Additiv: heterozyklisches Amin).

Eine toluolische Suspension des Triethylaminsalzes von 2-(2-Chlor-4-mesylbenzoyl)-cyclohexan-1,3-dion wird durch eine Umlagerung des korrespondierenden Enolesters 3-Oxo-cyclohex-1-enyl-(2-chlor-4-mesyl-benzoat) gewonnen (vgl. EP-A2-186 117). Der Enolester wird dabei durch Kopplung von 0,2 mol 2-Chlor-4-mesyl-benzoylchlorid mit einer äquimolaren Menge Cyclohexan-1,3-dion in Toluol in Anwesenheit eines Überschuss von Triethylamin als Base hergestellt. Diese toluolische Suspension wird für 30 Minuten bei 45°C mit 500 g verdünnter Natronlauge (4 Gew.-%) in Kontakt gebracht, um das Produkt als Natriumsalz gelöst in der wässrige Phase und befreit von Nebenprodukten zu erhalten. Nach Phasentrennung wird die wässrige Natriumenolatlösung bei 45°C gehalten. Die Bildung der stabilen Kristallstruktur (Modifikation II) des Triketons *per se* durch Fällung dieser wässrigen Natriumenolatlösung mit Salzsäure bedarf eines geeigneten Additivs, da die Bildung der metastabilen Phase (Modifikation I) im wässrigen System kinetisch bevorzugt ist. Dazu werden 5 g Pyridin zusammen mit 90 g dest. Wasser und 15 g Impfkristallen der Modifikation II (spezifische Oberfläche > 0,75 m$^2$/g) in einem Reaktionsgefäß vorgelegt, mit einem Impeller bei einem spezifischen Leistungseintrag von etwa 0,15 W/kg gerührt und auf 12°C temperiert. Nachdem der pH-Wert dieser Vorlage mit Salzsäure auf pH=2,7 eingestellt ist, wird zu dieser Suspension die wässrige Natriumenolatlösung sorgfältig mit einer Dosierrate von 1 g pro Minute zugetropft, während der pH-Wert durch kontrollierte Zugabe einer 37 Gew.-% Salzsäure konstant bei pH=2,7, die Temperatur konstant bei 12 °C und der Leistungseintrag durch den Rührer konstant bei 0,15 W/kg gehalten wird. Die beiden Eduktlösungen werden auf gegenüberliegenden Seiten auf die Oberfläche getropft. Das Produkt, das auf diese Art erhalten wird, wird filtriert und unter Vakuum bei einer Temperatur von 50°C getrocknet. Die Raman-Spetroskopie des Produktes zeigt die folgenden Peaks in [cm$^{-1}$]:
3175, 3096, 3083, 3067, 3002, 2965, 2948, 2918, 2900, 2874, 1660, 1589, 1543, 1458, 1422, 1397, 1358, 1337, 1322, 1310, 1283, 1266, 1247, 1228, 1152, 1117, 1050,992,958,935,845,779,752,718,676,660,613,594,578,560,513, 503,476, 460, 447, 429, 415, 371, 346, 328, 280, 236, 227,

welche mit denen der stabilen Modifikation II übereinstimmen (vgl. deutsche Patentanmeldung mit der Anmeldenummer 10152459.5).

### Beispiel 2

Herstellung der stabilen Kristallstruktur (Modifikation II) von 2-(2-Chlor-4-mesylbenzoyl)-cyclohexan-1,3-dion (Sulcotrione) durch direkte Fällung (Additiv: Ketone).

Das in Beispiel 1 beschriebene Verfahren wurde in analoger Weise auch mit Ketonen als Additiv durchgeführt, um 2-(2-Chlor-4-mesyl-benzoyl)-cyclohexan-1,3-dion in seiner stabilen Kristallstruktur (Modifikation II) direkt durch Fällungslaistallisation aus der korrespondierenden wässrigen Natriumenolatlösung zu erhalten. Die auf diese Weise erhaltene Kristallstruktur wurde anhand des charakteristischen Raman-Spektrums bestimmt, wie in Beispiel 1 ausgeführt. Die verwendeten Ketone, die eingesetzte Menge sowie das Ergebnis des Verfahrens sind in Tabelle I wiedergegeben.

**Tabelle I**

| Beispiel | Additiv | Menge des Additivs in Gramm | Kristallstruktur |
|---|---|---|---|
| 2(A) | Aceton | 5 | Mod. II |
| 2(B) | 2-Butanon | 5 | Mod. II |

### Beispiel 3

Herstellung der stabilen Kristallstruktur (Modifikation II) von 2-(2-Chlor-4-mesylbenzoyl)-cyclohexan-1,3-dion (Sulcotrione) durch direkte Fällung (Additiv: Alkohole).

Das in Beispiel 1 beschriebene Verfahren wurde in analoger Weise auch mit verschiedenen Alkoholen als Additiv durchgeführt, um 2-(2-Chlor-4-mesyl-benzoyl)-cyclohexan-1,3-dion in seiner stabilen Kristallstruktur (Modifikation II) direkt durch Fällungskristallisation aus der korrespondierenden wässrigen Natriumenolatlösung zu erhalten. Die auf diese Weise erhaltene Kristallstruktur wurde anhand des charakteristischen Raman-Spektrums bestimmt, wie in Beispiel 1 ausgeführt. Die verwendeten Alkohole, die eingesetzte Menge sowie das Ergebnis des Verfahrens sind in Tabelle II wiedergegeben.

**Tabelle II**

| Beispiel | Additiv | Menge des Additivs in Gramm | Kristallstruktur |
|---|---|---|---|
| 3(A) | Methanol | 5 | Mod. II |
| 3(B) | Ethanol | 5 | Mod. II |
| 3(C) | 2-Propanol | 5 | Mod. II |

### Beispiel 4

Herstellung der stabilen Kristallstruktur (Modifikation II) von 2-(2-Chlor-4-mesylbenzoyl)-cyclohexan-1,3-dion (Sulcotrione) durch direkte Fällung mit geringen Mengen Additiv.

Das in Beispiel 3 beschriebene Verfahren (Additiv: Ethanol) wurde in analoger Weise durchgeführt, um 2-(2-Chlor-4-mesyl-benzoyl)-cyclohexan-1,3-dion in seiner stabilen Kristallstruktur (Modifikation II) direkt durch Fällungskristallisation aus der korrespondierenden wässrigen Natriumenolatlösung zu erhalten, jedoch mit den in Tabelle III gezeigten Mengen Ethanol. Die auf diese Weise erhaltene Kristallstruktur wurde anhand des charakteristischen Raman-Spelctrums bestimmt, wie in Beispiel 1 ausgeführt. Die verwendeten Mengen sowie das Ergebnis des Versuchs sind in Tabelle m wiedergegeben.

**Tabelle III**

| Beispiel | Additiv | Menge des Additivs in Gramm | Kristallstruktur |
|---|---|---|---|
| 4(A) | Ethanol | 2,2 | Mod. II |
| 4(B) | Ethanol | 0,5 | Mod. II |

**Beispiel 5**

Verkürzung der Dosierzeiten durch Erhöhung der Menge des Additivs (250 g Methanol).

Die wässrige Natriumenolatlösung des 2-(2-Chlor-4-mesyl-benzoyl)-cyclohexan-1,3-dion wird auf dem in Beispiel 1 beschriebenen Weg hergestellt. Um die stabile Kristallstruktur (Modifikation II) des Triketons in kürzeren Dosierzeiten zu gewinnen, werden 250 g Methanol zusammen mit 22 g Impfkristallen der Modifikation II (spezifische Oberfläche > 0,75 m$^2$/g) in einem Reaktionsgefäß vorgelegt, gerührt mit einem Impeller bei einem spezifischen Leistungseintrag von etwa 0,15 W/kg und temperiert auf 12°C. Nachdem der pH-Wert dieser Vorlage mit Salzsäure auf pH=2,7 eingestellt wurde, wird zu dieser Suspension die wässrige Natriumenolatlösung sorgfältig mit einer Dosierrate von 4 g pro Minute zugetropft, während der pH-Wert durch kontrollierte Zugabe einer 37 Gew.-% Salzsäure konstant bei pH=2,7, die Temperatur konstant bei 12°C und der Leistungseintrag durch den Rührer konstant bei 0,15 W/kg gehalten wird. Die beiden Eduktlösungen werden gegenüberliegend auf die Oberfläche getropft. Das Produkt welches auf diese Art erhalten wird, wird filtriert und unter Vakuum bei einer Temperatur von 50°C getrocknet. Das in Beispiel 1 ausgeführte Raman-Spektnun kennzeichnet das auf diese Weise erhaltene Produkt als Modifikation II.

**Beispiel 6 - Vergleichsbeispiel**

Verkürzung der Dosierzeiten zur Fällung der stabilen Kristallmodifikation bei Einsatz geringer Mengen von Additiven durch Verwendung von Dispergiermaschinen.

Die wässrige Natriumenolatlösung des 2-(2-Chlor-4-mesyl-benzoyl)-cyclohexan-1,3-dion wird auf dem in Beispiel 1 beschriebenen Weg hergestellt. Um die stabile Kristallstruktur (Modifikation II) des Triketons in kürzeren Dosierzeiten zu gewinnen, werden 5 g Pyridin zusammen mit 90 g dest. Wasser und 15 g Impfkristalle der Modifikation II in einem Reaktionsgefäß vorgelegt und der pH-Wert mit Salzsäure auf pH=2,7 eingestellt. Diese Suspension wird daraufhin für zwei Minuten mit einer Dispergiermaschine behandelt. Nach dieser Behandlung wird die Suspension mit einem Impeller bei einem spezifischen Leistungseintrag von etwa 0,15 W/kg gerührt und auf 12°C temperiert, bevor zu dieser Suspension die wässrige Natriumenolatlösung sorgfältig mit einer Dosierrate von 2,5 g pro Minute zugetropft wird, während der pH-Wert durch kontrollierte Zugabe einer 37 Gew.-% Salzsäure konstant bei pH=2,7, die Temperatur konstant bei 12°C und der Leistungseintrag durch den Rührer konstant bei 0,15 W/kg gehalten wird. Die beiden Eduktlösungen werden auf gegenüberliegenden Seiten auf die Oberfläche getropft. Nachdem 320 g der wässrigen Natriumenolatlösung zugegeben wurden, wird die Suspension für weitere fünf Minuten mit der Dispergiermaschine behandelt. Dann wird die Dosierung der wässrigen Natriumenolatlösung mit einer Rate von 2,5 g pro Minute bei konstanter Temperatur und konstantem pH-Wert vervollständigt. Das Produkt welches auf diese Art erhalten wird, wird filtriert und unter Vakuum bei einer Temperatur von 50°C getrocknet. Das in Beispiel 1 ausgeführte Raman-Spektrum kennzeichnet das auf diese Weise erhaltene Produkt als Modifikation II.

**Beschreibung der Abbildungen**

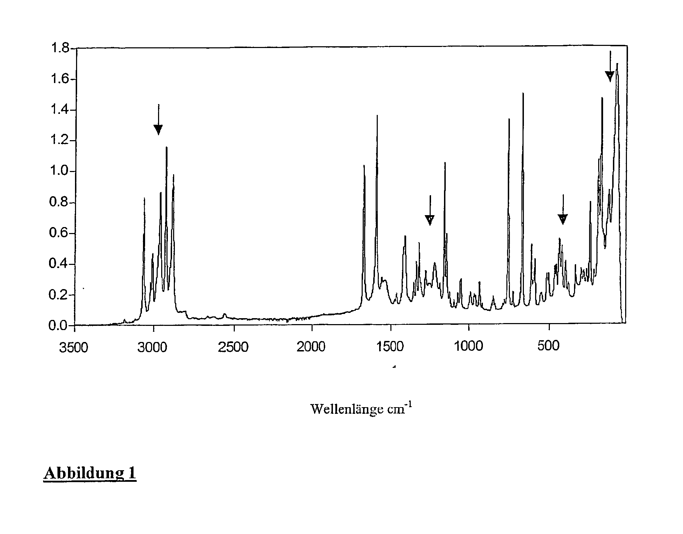

Abbildung 1    (Abb. 1) zeigt das Raman-Spektrum der Kristallmodifikation I von Sulcotrione. Die charakteristischen Peakmaxima sind mit Pfeilen gekennzeichnet.

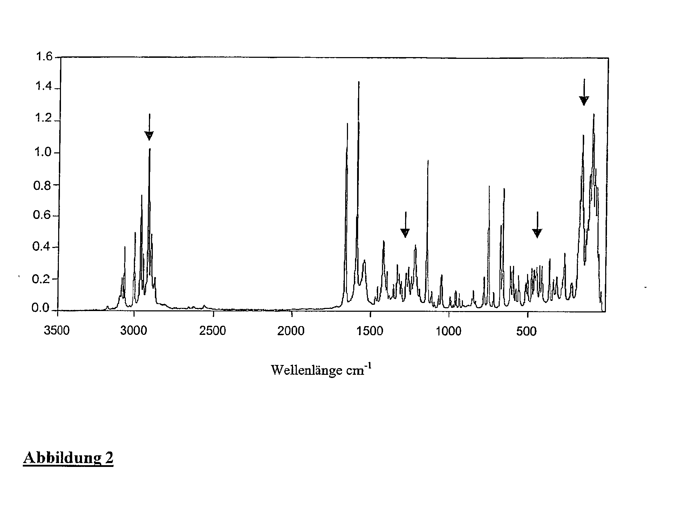

Abbildung 2    (Abb. 2) zeigt das Raman-Spektrum der Kristallmodifikation II von Sulcotrione. Die charakteristischen Peakmaxima sind mit Pfeilen gekennzeichnet.

**Patentansprüche**

1. Verfahren zur Herstellung einer spezifischen Kristallmodifikation von 2-(2-Chlor-4-mesyl-benzoyl)-cyclohexan-1,3-dion aus der wässrigen Lösung des Salzes von 2-(2-Chlor-4-mesyl-benzoyl)-cyclohexan-1,3-dion durch Ausfällen der spezifischen Kristallmodifikation mit einer Säure und mit einem wasserlöslichen, organischen Lösungsmittel aus der Gruppe der Ketone mit 3 bis 10 kohlenstoffatomen und Alkohole mit 1 bis 10 kohlenstoffatomen.

2. Verfahren gemäß Anspruch 1, worin das Keton aus der Gruppe umfassend Aceton und 2-Butanon stammt.

3. Verfahren gemäß Anspruch 1, worin der Alkohol aus der Gruppe umfassend Methanol, Ethanol und 2-Propanol stammt.

4.  Verfahren gemäß einem der Ansprüche 1 bis 3, worin der pH-Wert der wässrigen Lösung konstant bei 2,7 gehalten wird.

5.  Verfahren gemäß einem der Ansprüche 1 bis 4, wobei die Menge (w/w) des organischen Lösungsmittels weniger als 10 % bezogen auf die Menge der gefällten Substanz beträgt.

**Claims**

1.  Process for the production of a specific crystal modification of 2-(2-chloro-4-mesylbenzoyl)cyclohexane-1,3-dione from the aqueous solution of the salt of 2-(2-chloro-4-mesylbenzoyl)cyclohexane-1,3-dione by precipitating the specific crystal modification with an acid and with a water-soluble organic solvent from the group of the ketones with 3 to 10 carbon atoms and alcohols with 1 to 10 carbon atoms.

2.  Process according to Claim 1, wherein the ketone comes from the group comprising acetone and 2-butanone.

3.  Process according to Claim 1, wherein the alcohol comes from the group comprising methanol, ethanol and 2-propanol.

4.  Process according to any one of Claims 1 to 3, wherein the pH value of the aqueous solution is kept constant at 2.7.

5.  Process according to any one of Claims 1 to 4, wherein the amount (w/w) of the organic solvent is less than 10% of the amount of the precipitated substance.

**Revendications**

1.  Procédé pour la préparation d'une modification cristalline spécifique de la 2-(2-chloro-4-mésyl-benzoyl)-cyclohexane-1,3-dione à partir de la solution aqueuse du sel de 2-(2-chloro-4-mésyl-benzoyl)-cyclohexane-1,3-dione par précipitation de la modification cristalline spécifique par un acide et par un solvant organique hydrosoluble pris dans le groupe des cétones présentant de 1 à 10 atomes de carbone et des alcools présentant de 3 à 10 atomes de carbone.

2.  Procédé selon la revendication 1, où la cétone est prise dans le groupe comprenant l'acétone et la 2-butanone.

3.  Procédé selon la revendication 1, où l'alcool est pris dans le groupe comprenant le méthanol, l'éthanol et le 2-propanol.

4.  Procédé selon l'une des revendications 1 à 3, où la valeur de pH de la solution aqueuse est maintenue à un niveau constant de 2,7.

5.  Procédé selon l'une des revendications 1 à 4, où la quantité (en poids) du solvant organique est inférieure à 10 % par rapport à la quantité de la substance précipitée.

Wellenlänge cm$^{-1}$

**Abbildung 1**

Wellenlänge cm$^{-1}$

**Abbildung 2**